Europäisches Patentamt

European Patent Office

Office européen des brevets

(11) Veröffentlichungsnummer: **0 244 771 B1**

(12) **EUROPÄISCHE PATENTSCHRIFT**

(45) Veröffentlichungstag der Patentschrift: **30.12.92**

(51) Int. Cl.5: **C12N 9/96,** C12N 9/06, C12Q 1/26

(21) Anmeldenummer: **87106305.3**

(22) Anmeldetag: **30.04.87**

(54) **Stabilisierte NAD(P)H-abhängige lösliche Nitratreduktase, Verfahren zu ihrer Herstellung und ihre Verwendung.**

(30) Priorität: **02.05.86 DE 3614838**

(43) Veröffentlichungstag der Anmeldung:
**11.11.87 Patentblatt 87/46**

(45) Bekanntmachung des Hinweises auf die
Patenterteilung:
**30.12.92 Patentblatt 92/53**

(84) Benannte Vertragsstaaten:
**AT BE CH DE ES FR GB GR IT LI LU NL SE**

(56) Entgegenhaltungen:
DE-A- 1 930 059
FR-A- 2 084 751

THE PROCEEDINGS OF BIOTECHNOLOGY,
Band 1, 1984, Seiten 379-390; P. MONSAN et
al.: "Stabilization of enzyme activity"

METHODS OF ENZYMATIC ANALYSIS, Band
VII, Edition 3, 1985, Seiten 578-585; S. TANI-
GUCHI et al.: "Nitrate"

(73) Patentinhaber: **BOEHRINGER MANNHEIM GMBH**

Patentabteilung, Abt. E Sandhofer Strasse
112-132 Postfach 31 01 20
W-6800 Mannheim 31 Waldhof(DE)

(72) Erfinder: **Fischer, Stephan, Dr. rer. nat.
Moosstrasse 27
W-8120 Weilheim(DE)**
Erfinder: **Wurst, Bärbel
Hindenburgstrasse 12
W-8134 Pöcking(DE)**
Erfinder: **Beutler, Hans-Otto, Dr. rer. nat.
Zugspitzstrasse 28
W-8132 Tutzing(DE)**
Erfinder: **Kresse, Georg-Burkhard, Dr. rer. nat.
Alpspitzstrasse 6
W-8122 Penzberg(DE)**
Erfinder: **Brunner, Herwig, Dr.
Westendstrasse 11
W-8120 Weilheim(DE)**

(74) Vertreter: **Huber, Bernhard, Dipl.-Chem. et al Patentanwälte H. Weickmann, Dr. K. Fincke F.A. Weickmann, B. Huber Dr. H. Liska, Dr. J. Prechtel Kopernikusstrasse 9 Postfach 86 08 20 W-8000 München 86(DE)**

**Beschreibung**

Die Bestimmung von Nitrat gehört in der Lebensmittelchemie zu den elementaren analytischen Aufgaben. Eine große Zahl von analytischen Methoden ist bekannt, welche zur Ermittlung des Nitrat-Gehaltes in Lebensmitteln angewendet werden (siehe z.B. "Handbuch der Lebensmittelchemie II/2" (1967), Springer Verlag). Alle bekannten Analysenverfahren weisen jedoch den Nachteil einer sehr aufwendigen und umständlichen Durchführung auf. Die direkten Bestimmungsmethoden sind häufig unempfindlich und wenig spezifisch. Daher erfolgen Nitrat-Bestimmungen in der Praxis so, daß das Nitration zunächst reduziert und anschließend das Reduktionsprodukt nach einer Methode mit höherer Spezifität bestimmt wird. Entscheidend für die chemische Bestimmung von Nitrat ist, daß zunächst mit einem Reduktionsmittel zu Nitrit reduziert werden muß. Von der großen Zahl üblicher Reduktionsmittel hat sich zur Nitrat-Bestimmung lediglich die Anwendung eines Cadmium-Reduktors bewährt. Der Einsatz von Cadmium gilt jedoch in der Fachwelt als ausgesprochen problematisch, da bereits geringe Mengen von Cadmium toxisch und auch cancerogen sind.

Man hat sich bereits vielfach bemüht, das Cadmium durch ein anderes Reduktionsmittel zu ersetzen. Bisher ist jedoch kein geeignetes anderes Reduktionsmittel bekannt geworden. Daher besteht großes Interesse an einer spezifischen enzymatischen Methode.

Es sind fünf Enzyme bekannt, welche Nitrat in einer photometrisch meßbaren Reaktion umsetzen.

Die Nitrat-Reduktase (E.C. 1.9.6.1) reduziert Nitrat in Gegenwart von Ferrocytochrom, die Nitrat-Reduktase (E.C. 1.7.99.4) benötigt für die Nitrat-Umsetzung einen reduzierten Akzeptor - zum Beispiel Methylviologen -; ferner sind drei weitere Enzyme bekannt, welche Nitrat in Gegenwart von NADH, NADPH und NAD(P)H reduzieren.

Alle diese Enzyme sind in der Literatur ausführlich beschrieben worden. In etwa 200 Literaturstellen werden Herstellung aus verschiedenen tierischen, pflanzlichen und mikrobiellen Quellen, Möglichkeiten zur Reinigung, kinetische Eigenschaften und die physiologische Bedeutung in der lebenden Zelle beschrieben. Es liegen darunter zwei Publikationen vor, in denen auch eine Nitrat-Bestimmung mit Nitrat-umsetzenden Enzymen vorgeschlagen wird:

In Anal. Chem. 50, 1319, (1978) wird die Nitrat-Reduktion mit Hilfe einer Nitrat-Reduktase erwähnt; das gebildete Nitrit allerdings wird elektrochemisch nach Anwendung von Nitrit-Reduktase bestimmt.

Hamano et al in Agr. Biol.Chem. 47, 2427 (1983) verwenden zur Nitrat-Bestimmung in Fleisch- und Fischprodukten eine Nitrat-Reduktase (1.7.99.4), wobei das gebildete Nitrit in einer weitgehend spezifischen Diazotierungs-Reaktion gemessen wird.

Die Verwendung eines Enzyms, welches Nitrat in Gegenwart von NADH, NADPH oder NAD(P)H zu reduzieren vermag, zur Nitratbestimmung ist bisher nicht gelungen. Der Grund hierfür dürfte in der großen Instabilität des Enzyms liegen, wobei speziell die Eigenschaft des Enzyms, Nitrat mittels einem der erwähnten Coenzyme zu reduzieren, sehr instabil ist und in der Regel schon innerhalb einiger Stunden verloren geht, obwohl das Enzym andere, nicht direkt bestimmbare Reduktionsmittel immer noch benützen kann. Eine besondere Schwierigkeit lag aber auch darin, daß diese Enzyme auch eine Nitrit-Reduktaseaktivität aufweisen, die zu einer Weiterreduktion von gebildetem Nitrit führt und daher eine Endpunktbestimmung durch Messung von gebildetem Nitrit nicht zuläßt. In Z. Pflanzenphysiol. BD 66, S. 290-293 (1972) wird zwar bereits eine Nitratbestimmung mit Nitrat-Reduktase aus Spinatblättern beschrieben, wobei entweder oxidiertes NADH oder gebildetes Nitrit meßbar sein sollen, jedoch die Messung des Nitrits und nicht die von NAD empfohlen wird. Die Nacharbeitung dieses Verfahrens ergab jedoch keine brauchbaren Resultate.

Aufgabe der Erfindung war es daher, ein Verfahren zur Bestimmung von Nitrat zu schaffen, bei welchem die Nachteile und Schwierigkeiten der bisher beschriebenen Verfahren vermieden werden und mit hoher Genauigkeit innerhalb kurzer Zeit eine direkte Bestimmung der Umwandlung von NAD(P)H in NAD(P) als Maß für das von der Nitrat-Reduktase reduzierte Nitrat erfolgen kann. Ferner war es eine Aufgabe, ein für dieses Verfahren geeignetes Enzym und Reagenz zu schaffen.

Die Lösung dieses Problems gelingt erfindungsgemäß durch die Verwendung einer stabilisierten NAD-(P)H-abhängigen löslichen Nitrat-Reduktase vom assimilatorischen Typ, welche gekennzeichnet ist durch ein Molekulargewicht von etwa 90 000 D bei Elektrophorese in Anwesenheit von Natriumdodecylsulfat und eine Restaktivität nach drei Wochen bei 35°C von mehr als 60 %, und welche erhältlich ist, indem man eine Suspension des zerkleinerten Ausgangsmaterials in Tris-Weinsäurepuffer pH 7 - 8,5 herstellt, dieser lösliches Polyethylenimin in solcher Menge zusetzt, daß 10 bis 20 96 der Nitrat-Reduktaseaktivität in die auftretende Fällung übergehen, die Fällung abtrennt und den Überstand nach üblichen biochemischen Fraktionierungsmethoden im oben genannten Aufschlußpuffer aufarbeitet, die erhaltene Enzymlösung dialysiert und in einem zwitterionischen Puffer lyophilisiert.

3

EP 0 244 771 B1

Das erfindungsgemäß verwendete Enzym E.C. 1.6.6.2 weist eine wesentlich verbesserte Stabilität seiner Reduktaseaktiviät mit NAD(P)H als Coenzym auf und kann in Gegenwart geeigneter Puffer nicht nur in lyophilisiertem, sondern auch in rekonstituiertem Zustand in wäßriger Lösung zur Nitratbestimmung eingesetzt werden, ohne daß eine störende Nitrit-Reduktasewirksamkeit auftritt.

Die Herstellung des erfindungsgemäßen stabilisierten NAD(P)H-abhängigen löslichen Nitrat-Reduktasepräparates vom assimilatorischen Typ aus biologischem Material erfolgt durch Zerkleinerung, Aufschluß in neutralem bis schwach alkalischem Medium und Fraktionierung in Gegenwart eines Komplexbildners und eines Merkaptans und ist dadurch gekennzeichnet, daß man eine Suspension des zerkleinerten Ausgangsmaterials in Tris-Weinsäurepuffer pH 7 - 8,5 herstellt, dieser lösliches Polyethylenimin in solcher Menge zusetzt, daß 10 bis 20 % der Nitrat-Reduktaseaktivität in die auftretende Fällung übergehen, daß man die Fällung abtrennt und den Überstand nach üblichen biochemischen Fraktionierungsmethoden im oben genannten Aufschlußpuffer aufarbeitet, die erhaltene Enzymlösung dialysiert und in einem zwitterionischen Puffer lyophilisiert.

Wesentlich für die Erzielung der gewünschten Eigenschaften ist die Verwendung des speziellen Puffers, die Polyethyleniminfraktionierung unter Bedingungen, die keine quantitative Zurückhaltung des Enzyms in der gelösten Phase ergeben, sowie die Lyophilisierung des erhaltenen, angereicherten, stabilisierten Enzyms in einem zwitterionischen Puffer.

Der Aufschlußpuffer auf Basis von Tris-Weinsäure weist einen pH-Wert zwischen 7 und 8,5 und eine zweckmäßige Konzentration zwischen 10 und 100 mmol/l auf. Bevorzugt wird eine 30 bis 70 mmolare Tris-Weinsäurepufferlösung mit einem pH-Wert zwischen 7,2 und 8,0 verwendet. Als Ausgangsmaterial können an sich die bekannten biologischen Materialien, die NAD(P)H-abhängige Nitrat-Reduktasen vom assimilatorischen Typ enthalten, verwendet werden. Bevorzugt wird von einem Schimmelpilz, insbesondere Aspergillus Niger, ausgegangen. Besonders bevorzugt wird Aspergillus sp. DSM 1729 oder Aspergillus nidulans DSM 63358 als Ausgangsmaterial.

Vorzugsweise setzt man dem verwendeten Puffer einen Proteaseinhibitor zu, wobei die dem Fachmann bekannten Proteaseinhibitoren verwendet werden können. Bevorzugt werden als Proteaseinhibitoren Benzamidin oder/und PMSF verwendet. Weiter wird dem Puffer vorzugsweise Zucker oder Zuckeralkohol zugesetzt, wobei die Konzentration bis 20 % (G/V) betragen kann. Besonders bevorzugt werden als Zucker Saccharose und als Zuckeralkohol Glycerin verwendet, jedoch können auch andere Zucker, wie Mannose usw., oder Zuckeralkohole wie Hexite, Pentite und dergleichen, eingesetzt werden.

Bei der Reinigung des Enzyms wird in üblicher Weise in Gegenwart eines Komplexbildners, wie z.B. EDTA, und eines Merkaptans, wie Glutathion oder Merkaptoethanol, gearbeitet. Als vorteilhaft erwies sich auch ein Zusatz von FAD.

Zur Feinreinigung nach üblichen biochemischen Fraktionierungsmethoden wie Ammonsulfat-Fraktionierung, Austauscherchromatographie oder/und Molekularsieb-Fraktionierung können diese an sich in beliebiger Weise eingesetzt werden. Wesentlich ist hierbei aber, daß stets in Gegenwart des Aufschlußpuffers, wie oben angegeben, gearbeitet wird. Vorzugsweise setzt man dem Überstand der Polyethyleniminfällung so viel schwach alkalischen Anionenaustauscher zu, daß noch etwas Nitratreduktaseaktivität in der Lösung verbleibt, also nicht alle Enzymaktivität an den Austauscher gebunden wird, und eluiert dann den Austauscher durch Erhöhung der Ionenkonzentration. Geeignete schwach alkalische Anionenaustauscher sind beispielsweise solche mit Diethylaminoethanol-Gruppen, gebunden an geeignete Trägermaterialien, wie Agarose, Cellulose und dergleichen.

Zur chromatographischen Anreicherung erwies sich insbesondere eine aromatisch substituierte, vernetzte Agarose als geeignet, und ein derartiger Anreicherungsschritt wird daher im Rahmen der Erfindung bevorzugt. Besonders bevorzugt ist eine Reinigung über Phenylsepharose.

Zur Entfernung von Verunreinigungen ist es außerdem zweckmäßig, vor oder nach der Anionenaustauscherbehandlung oder/und der Chromatographie das Enzym durch Zusatz von Ammoniumsulfat auszufällen und die Fällung dann wieder in Aufschlußpuffer zu lösen für die weitere Bearbeitung.

Andere Anreicherungsschritte, die zur Gewinnung des erfindungsgemäßen Enzyms angewendet werden können, sind Affinitätschromatographie an Cytochrom C enthaltender Agarose, z.B. Cytochrom C aus Pferdeherz gekoppelt an Sepharose CL6B$^R$, an Farbliganden wie z.B. Blue-Sepharose oder FAD-Sepharose. Besonders bevorzugt wird jedoch die Reinigung an Phenylsepharose.

Das für das Verfahren der Erfindung bevorzugt verwendete Enzym aus Schimmelpilzen weist in nativem Zustand ein MG von ca. 200 000 auf, enthält FAD und Molybdän und besitzt eine $K_M$ gegenüber Nitrat von etwa $3,2 \times 10^{-4}$ M. Die Bestimmung des Molekulargewichts erfolgt über Gelfiltration.

Als zwitterionischer Puffer wird im Rahmen der Erfindung ein GOOD-Puffer bevorzugt. Besonders bevorzugt wird als GOOD-Puffer wiederum HEPES-Puffer. Zur Durchführung der Lyophilisierung wird das Enzym entweder ausgefällt, z.B. durch Ammoniumsulfatzusatz, und dann im zwitterionischen Puffer aufge-

4

nommen, oder man dialysiert die Lösung des Enzyms im Aufschlußpuffer gegen den zwitterionischen Puffer. Der zwitterionische Puffer wird zweckmäßig in 0,01 bis 0,2 molarer Konzentration, besonders bevorzugt in 0,02 bis 0,1 molarer Konzentration eingesetzt. Er enthält zweckmäßig kleine Mengen Komplexbildner, beispielsweise 0,1 bis 50 mM EDTA, geringe Mengen FAD, insbesondere 1 bis 30 $\mu$M, und eine Sulfhydryl-Verbindung, zweckmäßig in einer Konzentration von 0,5 bis 10 mM, und wird mit einer geeigneten Base, wie z.B. Natronlauge, auf einen pH-Wert zwischen 6,5 und 9, vorzugsweise 7 bis 8, eingestellt.

Das erfindungsgemäße Enzym besitzt ein pH-Optimum um 7,5 und ist bei 35°C in 50 mM Kaliumphosphat-Puffer pH 7,5 bei 10 minütiger Inkubation stabil.

Die Substratspezifität beträgt für Nitrat 100 %, für Nitrit 0,12 %, NAD/P/H 100 % und NADH 13 %.

Inhibitoren sind Nitrit bei über $10^{-4}$ M (Produkthemmung), $Mn^{2+}$, $Fe^{2+}$, $Cu^{2+}$, $Fe^{3+}$ (in Abwesenheit von EDTA), $CN^-$ und Azid.

Für die Verwendung zur Nitratbestimmung wird die erfindungsgemäße Nitrat-Reduktase, die in lyophilisiertem Zustand vorliegt und einen zwitterionischen Puffer, wie oben näher beschrieben, enthält, in einem Stickstoffheterocycluspuffer mit einem pH-Wert zwischen 6,5 und 9 aufgelöst und enthält daher in rekonstituiertem Zustand sowohl den zwitterionischen Puffer als auch den Stickstoffheterocycluspuffer.

Demzufolge ist ein erfindungsgemäßes Reagenz zur Bestimmung von Nitrat in gelöster Form gekennzeichnet durch einen Gehalt an Nitrat-Reduktase, wie oben definiert, NAD(P)H, zwitterionischen Puffer pH 7 bis 9 und Stickstoffheterocycluspuffer pH 6,5 bis 9. Zur Durchführung der Nitratbestimmung wird lediglich das rekonstituierte Reagenz der nitrathaltigen Probe zugesetzt und die Oxidation von NAD(P)H durch Messung der Abnahme des reduzierten oder Zunahme des oxidierten Coenzyms bestimmt. Als Stickstoffheterocycluspuffer wird bevorzugt Imidazolpuffer, Pyrazolpuffer oder Pyridinpuffer, wobei Imidazolpuffer besonders bevorzugt ist. Der bevorzugte pH-Wert beträgt 7,0 bis 7,8 und die bevorzugte Pufferkonzentration im rekonstituierten löslichen Reagenz 20 bis 50 mmol/l.

Das erfindungsgemäße Reagenz enthält als weiteren Bestandteil vorzugsweise Superoxiddismutase (SOD), zweckmäßigerweise in einer Menge zwischen 10 und 1000 Einheiten/ml gelöstem Reagenz. Außerdem enthält es vorzugsweise mehrere oder alle vorstehend für den Aufschlußpuffer genannten Zusatzstoffe in den dort angegebenen Mengen.

Die folgenden Beispiele erläutern die Erfindung weiter:

**Beispiel 1**

A) Aufschluß

9 kg abfiltrierte Biomasse von Aspergillus spez. DSM 1729 in 50 Ltr. eiskaltem Puffer, bestehend aus 0,05 M Tris/Weinsäurepuffer, enthaltend 5 mM EDTA, 5 $\mu$M FAD, 2 mM Mercaptoethanol, pH 7,5, aufnehmen. Die Suspension homogenisieren und mittels Hochdruckdispersion (600 bar) aufschließen. Zur aufgeschlossenen Biomasse 10 % Glycerin, 10 $\mu$g PMSF/Ltr. Flüssigkeit und 15 $\mu$g Benzamidin/Ltr. Flüssigkeit zusetzen und den pH-Wert mit 1 M Tris-Base auf 7,5 nachstellen.

| Vol.: | 70 Ltr. |
|---|---|
| SU Aktivität: | 75 KU |
| SA ca. 0,12 U/mg | |

B) Polymin-P-Abtrennung

Die Füssigkeit aus Stufe A unter Rühren nach Vorproben (mit je 4 %, 6 %, 8 %, 10 % einer 1 %igen Polymin P-Lösung, pH 7,0) mit soviel Polymin P versetzen, daß 85 % der Aktivität im Überstand verbleiben. Nach 15 Min. Rühren filtrieren, den Niederschlag verwerfen, das Filtrat mit 7 mg PMSF und 15 mg Benzamidin pro Liter Flüssigkeit versetzen.

| Vol.: | 70 Ltr. |
|---|---|
| SU Aktivität: | 70 KU |

C) DEAE-Sephadex-batch

In das Filtrat von Stufe B nach vorproben (2 %, 3 %, 4 %) soviel DEAE-Sephadex einrühren, daß mehr als 10 % der Aktivität im Überstand verbleiben. Den Austauscher abfiltrieren und mit 3 Ltr. Tris/Weinsäure-Puffer, enthaltend 5 mM EDTA, 5 $\mu$M FAD und 2 mM Mercaptoethanol, pH 7,5, waschen.

Die Elution erfolgt durch Zugabe von 0,5 M NaCl zum Waschpuffer (mit insgesamt 3 x 3 Ltr.). Die Eluate vereinigen, mit 7 mg PMSF und 15 mg Benzamidin pro Liter versetzen und kühl stellen.

| Vol. : | ca. 9 Ltr. | SU Aktivität: | 65 KU |
|---|---|---|---|

D) Ammoniumsulfat-Fällung

Das Eluat von Stufe C nach Vorproben (2,0 M, 2,2 M, 2,4 M) mit festem Ammoniumsulfat versetzen, bis weniger als 5 % der Aktivität im Überstand verbleiben. Den Niederschlag zentrifugieren, den blanken Überstand verwerfen.

E) Phenylsepharose-Chromatographie

Den AS-Niederschlag von Stufe D mit 1 Ltr. Tris/Weinsäurepuffer (0,05 M), enthaltend 5 mM EDTA, 5 $\mu$M FAD, 2 mM Mercaptoethanol, 10 % v/v Glycerin und 1 M Ammoniumsulfat pH 7,5, aufnehmen und mit 7 mg PMSF und 15 mg Benzamidin pro Liter versetzen, evtl. Trübung abzentrifugieren.

| Vol.: | 1,4 Ltr. | SU Aktivität: | 45 KU |
|---|---|---|---|
| | | SA 1,7 U/mg | |

Die Enzymlösung auf eine 2 Ltr.-Phenylsepharose-Säule aufziehen (Säule 10 x 28 cm, äquilibriert mit 0,05 M Tris/Weinsäure-Puffer, pH 7,5, enthaltend 5 mM EDTA, 5 $\mu$M FAD, 2 mM Mercaptoäthanol, 10 % (v/v) Glycerin und 1 M Ammoniumsulfat). Die Waschung erfolgt mit 3 Ltr. des oben genannten Puffers.

Eluiert wird mit einem Gradienten, bestehend aus

A:  2 Ltr. Puffer wie Waschpuffer, enthaltend jedoch 0,5 M Ammoniumsulfat

B:  2 Ltr. Puffer wie Waschpuffer, enthaltend 5 mM Amnoniumsulfat

Fraktionen mit Aktivität größer 5 U/ml vereinigen und mit 7 mg PMSF und 15 mg Benzamidin pro Liter versetzen.

| Vol.: | 2,5 Ltr. | SU AKtivität: | 36 KU |
|---|---|---|---|
| | | SA 4 U/mg | |

F) Ammoniumsulfat-Konzentrierung und Dialyse

Das Eluat aus Stufe E mit festem Ammoniumsulfat bis 2,4 M ausfällen, zentrifugieren, den blanken Überstand verwerfen. Den Niederschlag in 250 ml 0,05 M Tris/Weinsäure-Puffer, enthaltend 5 mM EDTA, 5 $\mu$M FAD, 2 mM Mercaptoaethanol und 10 % Glycerin, pH 7,5, aufnehmen, mit 7 mg PMSF und 15 mg Benzamidin pro Liter versetzen.

G) Dialyse

Die Lösung aus Stufe F gegen 3 x 5 Ltr. 0,05 M HEPES-Puffer, enthaltend 5 mM EDTA, 5 $\mu$M FAD, 2 mM Mercaptoaethanol, pH 7,5 (eingestellt mit 1 N NaOH), dialysieren.

Das Dialysat mit Dialysepuffer auf 60 U/ml verdünnen, mit 95 $\mu$M FAD und 2 mM GSH aufstocken und mit 80 mg Ficoll pro ml Enzymlösung versetzen. Den pH-Wert mit 1 M Tris-Base auf 7,5 einstellen, keimarm filtrieren, einfrieren und lyophilisieren.

Ausbeute:             ca. 50 g Lyophilisat
SU Aktivität:         25 KU = 30 % vom Ausgang 1 g Protein
Aktivität:            0,5 U/mg Lyophilisat
Spezifische Aktivität: 10 U/mg Protein
Fremdaktivitäten:     NADPH-Oxidase 0,7 %
                      NADPH-abhängige ADH: 0,8 %
                      Nitritreduktase: 0,2 %
Lagerung:             -20 °C

**Beispiel 2**

Nitratbestimmung

Testbedingungen:

Wellenlänge: (Hg) 365 nm, (Hg) 334 nm, 339 nm; Schichtdicke 1 cm; Testvolumen 2,75 ml; Temperatur: 20 - 25°C; Messung gegen Luft.

Messung:

| In Küvetten pipettieren im Test | Probe | Leerwert | Konzentrationen |
|---|---|---|---|
| K-Phosphat(0,1mol/l;pH7) | 1,00ml | 1,00 ml | Phosphat     35 mmol/l |
| FAD ($10^{-4}$ mol/l) | 0,10ml | 0,10 ml | FAD          3,6 µmol/l |
| NADPH (c=10mg/ml) | 0,05ml | 0,05 ml | NADPH        175 µmol/l |
| Probe (z.B. $KNO_3$) | 0,05ml | -- | $NO_3^-$, bis zu 140 µmo. |
| Wasser | 1,50ml | 1,55 ml | |

mischen, ca. 3 min bei Raumtemperatur stehen lassen; Messung von

| Nitrat-Reduktase (∿6 U/ml) | 0,05ml | 0,05 ml | NR    ca. 115 U/l |
|---|---|---|---|

mischen; ca. 20-30 min bei Raumtemperatur stehen lassen; $E_2$ messen

Extinktionsdifferenzen $(E_1 - E_2)$ von Probe und Leerwert berechnen.

$$\Delta E_{(Nitrat)} = (E_1 - E_2)_{Probe} - (E_1 - E_2)_{Leerwert}$$

Berechnung

$$c\ [g/l] = \frac{V \times MG \times \Delta E}{\varepsilon \times d \times v \times 1000}$$

für Nitrat:

c = Konzentration von Nitrat in der Probelösung (g/l)
V = Testvolumen in ml (hier: 2,75 ml)
MG = Molekulargewicht (hier: Nitrat = 62,00 g/mol)
$\Delta E$ = Extinktion, s. oben
$\epsilon$ = Extinktionskoeffizient bei: 334 nm (Hg) = 6,18 1 x mmol$^{-1}$ x cm$^{-1}$ 339 nm = 6,3 1 x mmol$^{-1}$ x cm$^{-1}$ 365 nm (Hg) = 3,5 1 x mmol$^{-1}$ x cm$^{-1}$
d = Schichtdicke der Küvette (hier: 1 cm)
v = Probevolumen (hier: 0,05 ml)

$$c = \frac{2,75 \times 62,0 \times \Delta E}{\varepsilon \times 1 \times 0,05 \times 1000} = \frac{3,410}{\varepsilon} \times \Delta E\ [g\ Nitrat\ Probelösung$$

7

**Beispiel 3**

Nitratbestimmung

Testbedingungen:

Wellenlänge: (Hg) 365 nm, (Hg) 334 nm, 339 nm; Schichtdicke 1 cm; Testvolumen 2,77 ml; Temperatur: 20-25°C; Messung gegen Luft.

Messung

| In Küvetten pipettieren | Probe | Leerwert | Konzentrationen im Test |
|---|---|---|---|
| Imidazol(0,1mol/1;pH7,3) | 1,00ml | 1,00ml | Imidazol 35 mmol/1 |
| FAD (10$^{-4}$mol/1) | 0,10ml | 0,10ml | FAD 3,6μmol/1 |
| NADPH (c=10mg/ml) | 0,05ml | 0,05ml | NADPH 175μmol/1 |
| Superoxiddismutase | | | |
| (6mg/ml) | 0,02ml | 0,02ml | SOD 217 U/1 |
| Probe (z.B. KNO$_3$) | 0,05ml | -- | NO$_3^-$, bis zu 140μmol/1 |
| Wasser | 1,50ml | 1,55ml | |

mischen, ca. 3 min. bei Raumtemperatur stehen lassen; Messung von $E_1$

| | | | |
|---|---|---|---|
| Nitrat-Reduktase | | | |
| (∼6U/ml) | 0,05ml | 0,05ml | NR ca. 115 U/1 |

mischen; ca 30-40 min. bei Raumtemperatur stehen lassen; $E_2$ messen.

Extinktionsdifferenzen ($E_1 - E_2$) von Probe und Leerwert berechnen.

$$\Delta E_{(Nitrat)} = (E_1 - E_2)_{Probe} - (E_1 - E_2)_{Leerwert}$$

Berechnung

$$c\left[gNO_3/1 \ Probelösung\right] = \frac{V \times MG \times \Delta E}{\varepsilon \times d \times v \times 1000}$$

für Nitrat:

c =     Konzentration von Nitrat in der Probelösung (g/l)
V =     Testvolumen in ml (hier: 2,77 ml)
MG =     Molekulargewicht (hier: Nitrat = 62,00 g/mol)
ΔE =     Extinktionsdifferenz, s. oben
ε =     Extinktionskoeffizient von NADPH bei:

8

334 nm (Hg) = 6,18 1 x mmol$^{-1}$ x cm $^{-1}$
339 nm = 6,3 1 x mmol$^{-1}$ x cm $^{-1}$
365 nm (Hg) = 3,5 1 x mmol$^{-1}$ x cm $^{-1}$

d = Schichtdicke der Küvette (hier: 1 cm)
v = Probevolumen (hier: 0,05 ml)

$$c = \frac{2,77 \times 62,00 \times \Delta E}{\varepsilon \times 1 \times 0,05 \times 1000} = \frac{3,435}{\varepsilon} \times \Delta E$$

$$[g \ Nitrat/l \ Probelösung]$$

**Patentansprüche**
**Patentansprüche für folgende Vertragsstaaten : BE, CH, DE, FR, GB, IT, LI, LU, NL, SE**

1. Stabilisierte NAD(P)H-abhängige lösliche Nitratreduktase vom assimilatorischen Typ, **gekennzeichnet durch** ein Molekulargewicht von etwa 90 000 D bei Elektrophorese in Anwesenheit von Natriumdodecylsulfat und eine Restaktivität nach drei Wochen bei 35°C von mehr als 60%, erhältlich, indem man eine Suspension NAD(P)H-abhängige Nitratreduktase vom assimilatorischen Typ enthaltenden zerkleinerten Ausgangsmaterials in Tris-Weinsäurepuffer pH 7 - 8,5 herstellt, dieser lösliches Polyethylenimin in solcher Menge zusetzt, daß 10 bis 20% der Nitratreduktaseaktivität in die auftretende Fällung übergehen, die Fällung abtrennt und den Überstand nach üblichen biochemischen Fraktionierungsmethoden im oben genannten Aufschlußpuffer aufarbeitet, die erhaltene Enzymlösung dialysiert und in einem zwitterionischen Puffer lyophilisiert.

2. Verfahren zur Herstellung einer stabilisierten, NAD(P)H-abhängigen löslichen Nitratreduktase vom assimilatorischen Typ aus biologischem Material durch Zerkleinerung, Aufschluß in neutralem bis schwach alkalischem Medium und Fraktionierung in Gegenwart eines Komplexbildners und eines Merkaptans, insbesondere nach Anspruch 1, **dadurch gekennzeichnet**, daß man eine Suspension NAD(P)H-abhängige Nitratreduktase vom assimilatorischen Typ enthaltenden zerkleinerten Ausgangsmaterials in Tris-Weinsäurepuffer pH 7 - 8,5 herstellt, dieser lösliches Polyethylenimin in solcher Menge zusetzt, daß 10 bis 20% der Nitratredukaseaktivität in die auftretende Fällung

3. Verfahren nach Anspruch 2, **dadurch gekennzeichnet,** daß man dem Puffer einen Proteaseinhibitor zusetzt.

4. Verfahren nach Anspruch 2 oder 3, **dadurch gekennzeichnet,** daß man dem Puffer FAD zusetzt.

5. Verfahren nach Anspruch 2, **dadurch gekennzeichnet,** daß man dem Puffer Zucker oder Zuckeralkohol zusetzt.

6. Verfahren nach einem der Ansprüche 2 bis 5, **dadurch gekennzeichnet,** daß man dem Überstand der Polyethyleniminfällung soviel schwachalkalischen Anionenaustauscher zusetzt, daß noch Nitratreduktaseaktivität in der Lösung verbleibt und daß man den Austauscher dann mit erhöhter Ionenkonzentration eluiert.

7. Verfahren nach einem der Ansprüche 2 bis 6, **dadurch gekennzeichnet,** daß man die Enzymlösung über aromatisch substituierte vernetzte Agarose chromatographiert. übergehen, die Fällung abtrennt und den Überstand nach üblichen biochemischen Fraktionierungsmethoden im oben genannten Aufschlußpuffer aufarbeitet, die erhaltene Enzymlösung dialysiert und in einem zwitterionischen Puffer lyophilisiert.

8. Verfahren nach einem der Ansprüche 2 bis 7, **dadurch gekennzeichnet**, daß man vor oder nach der Anionenaustauscherbehandlungoder/und der Chromatographie das Enzym mit Ammonsulfat fällt und dann erneut in Tris-Weinsäurepuffer pH 7 - 8,5 löst.

9. Reagenz zur Bestimmung von Nitrat, **gekennzeichnet durch** einen Gehalt an Nitratreduktase gemäß Anspruch 1, NAD(P)H, zwitterionischem Puffer pH 7 bis 9, Stickstoffheterocycluspuffer pH 6,5 bis 9.

10. Reagenz nach Anspruch 9, daß der zwitterionische Puffer ein GOOD-Puffer ist.

11. Reagenz nach Anspruch 9 oder 10, **dadurch gekennzeichnet,** daß der Stickstoffheterocycluspuffer ein Imidazolpuffer, Pyrazolpuffer oder Pyridinpuffer ist.

12. Reagenz nach einem der Ansprüche 9 bis 11, **dadurch gekennzeichnet,** daß der pH-Wert 7,0 bis 7,8 beträgt und die Pufferkonzentration im rekonstituierten gelösten Reagenz 20 bis 50 mmol ist.

13. Reagenz nach einem der Ansprüche 9 bis 12, **dadurch gekennzeichnet,** daß es Superoxiddismutase enthält.

14. Reagenz nach einem der Ansprüche 9 bis 13, **dadurch gekennzeichnet,** daß es wenigstens einen Zusatzstoff gemfäß den Ansprüchen 3, 4 oder 5 enthält.

**Patentansprüche für folgende Vertragsstaaten : AT, ES, GR**

1. Verfahren zur Herstellung einer stabilisierten, NAD(P)H-abhängigen löslichen Nitratreduktase vom assimilatorischen Typ aus biologischem Material durch Zerkleinerung, Aufschluß in neutralem bis schwach alkalischem Medium und Fraktionierung in Gegenwart eines Komplexbildners und eines Merkaptans,
dadurch gekennzeichnet, daß man eine Suspension NAD(P)H-abhängige Nitratreduktase vom assimilatorischen Typ enthaltenden zerkleinerten Ausgangsmaterials in Tris-Weinsäurepuffer pH 7 - 8,5 herstellt, dieser lösliches Polyethylenimin in solcher Menge zusetzt, daß 10 bis 20% der Nitratredukaseaktivität in die auftretende Fällung übergehen, die Fällung abtrennt und den Überstand nach üblichen biochemischen Fraktionierungsmethoden im oben genannten Aufschlußpuffer aufarbeitet, die erhaltene Enzymlösung dialysiert und in einem zwitterionischen Puffer lyophilisiert und das Enzym mit einem Molekulargewicht von etwa 90 000 D bei Elektrophorese in Anwesenheit von Natriumdodecylsulfat und einer Restaktivität nach drei Wochen bei 35°C von mehr als 60% gewinnt.

2. Verfahren nach Anspruch 1, **dadurch gekennzeichnet,** daß man dem Puffer einen Proteaseinhibitor zusetzt.

3. Verfahren nach Anspruch 1, oder 2, **dadurch gekennzeichnet,** daß man dem Puffer FAD zusetzt.

4. Verfahren nach Anspruch 1, **dadurch gekennzeichnet,** daß man dem Puffer Zucker oder Zuckeralkohol zusetzt.

5. Verfahren nach einem der Ansprüche 1 bis 4 **dadurch gekennzeichnet,** daß man dem Überstand der Polyethyleniminfällung soviel schwachalkalischen Anionenaustauscher zusetzt, daß noch Nitratreduktaseaktivität in der Lösung verbleibt und daß man den Austauscher dann mit erhöhter Ionenkonzentration eluiert.

6. Verfahren nach einem der Ansprüche 1 bis 5, **dadurch gekennzeichnet,** daß man die Enzymlösung über aromatisch substituierte vernetzte Agarose chromatographiert.

7. Verfahren nach einem der Ansprüche 1 bis 6 dadurch gekennzeichnet, daß man vor oder nach der Anionenaustauscherbehandlung oder/und der Chromatographie das Enzym mit Ammonsulfat fällt und dann erneut in Tris-Weinsäurepuffer pH 7 - 8,5 löst.

8. Reagenz zur Bestimmung von Nitrat, **gekennzeichnet durch** einen Gehalt an gemäß Anspruch 1 enthaltener Nitratreduktase NAD(P)H, zwitterionischem Puffer pH 7 bis 9, Stickstoffheterocycluspuffer ph 6,5 bis 9.

9. Reagenz nach Anspruch 8, daß der zwitterionische Puffer ein GOOD-Puffer ist.

**10.** Reagenz nach Anspruch 8 oder 9, **dadurch gekennzeichnet,** daß der Stickstoffheterocycluspuffer ein Imidazolpuffer, Pyrazolpuffer oder Pyridinpuffer ist.

**11.** Reagenz nach einem der Ansprüche 8 bis 10 **dadurch gekennzeichnet,** daß der pH-Wert 7,0 bis 7,8 beträgt und die Pufferkonzentration im rekonstituierten gelösten Reagenz 20 bis 50 mmol ist.

**12.** Reagenz nach einem der Ansprüche 8 bis 11 **dadurch gekennzeichnet**, daß es Superoxiddismutase enthält.

**13.** Reagenz nach einem der Ansprüche 8 bis 12 **dadurch gekennzeichnet**, daß es wenigstens einen Zusatzstoff gemäß den Ansprüchen 2, 3 oder 4 enthält.

**Claims**
**Claims for the following Contracting States : BE, CH, DE, FR, GB, IT, LI, LU, NL, SE**

**1.** Stabilised, NAD(P)H-dependent, soluble nitrate reductase of the assimilatory type, characterised by a molecular weight of about 90,000 D in the case of electrophoresis in the presence of sodium dodecyl sulphate and a residual activity after 3 weeks at 35°C of more than 60%, obtainable in that one prepares a suspension of the comminuted starting material, containing NAD(P)H-dependent nitrate reductase of the assimilatory type, in tris-tartaric acid buffer pH 7 - 3.5, adds to this soluble polyethyleneimine in such an amount that 10 to 20% of the nitrate reductase activity passes into the precipitation obtained, separates off the precipitate and works up the supernatant according to usual biochemical fractionation methods in the above-mentioned digestion buffer, dialyses the enzyme solution obtained and lyophilises in a zwitterionic buffer.

**2.** Process for the preparation of a stabilised, NAD(P)H-dependent, soluble nitrate reductase of the assimilatory type from biological material by comminution, digestion in neutral to weakly alkaline medium and fractionation in the presence of a complex former and of a mercaptan, especially according to claim 1, characterised in that one prepares a suspension of the comminuted starting material. containing NAD(P)H-dependent nitrate reductase of the assimilatory type, in tris-tartaric acid buffer pH 7 - 8.5, adds to this soluble polyethyleneimine in such an amount that 10 to 20% of the nitrate reductase activity passes over into the precipitate obtained, separates off the precipitate and works up the supernatant according to usual biochemical fractionation methods in the above-mentioned digestion buffer, dialyses the enzyme solution obtained and lyophilises in a zwitterionic buffer.

**3.** Process according to claim 2, characterised in that one adds a protease inhibitor to the buffer.

**4.** Process according to claim 2 or 3, characterised in that one adds FAD to the buffer.

**5.** Process according to claim 2, characterised in that one adds sugar or sugar alcohol to the buffer.

**6.** Process according to one of claims 2 to 5, characterised in that one adds to the supernatant of the polyethyleneimine precipitation so much weakly alkaline anion exchanger that nitrate reductase activity still remains in the solution and that one then elutes the exchanger with increased ion concentration.

**7.** Process according to one of claims 2 to 6, characterised in that one chromatographs the enzyme solution over aromatic-substituted, cross-linked agarose.

**8.** Process according to one of claims 2 to 7, characterised in that, before or after the anion exchanger treatment and/or the chromatography, one precipitates the enzyme with ammonium sulphate and then again dissolves in tris-tartaric acid buffer pH 7 - 8.5.

**9.** Reagent for the determination of nitrate, characterised by a content of nitrate reductase according to claim 1, NAD(P)H, a zwitterionic buffer pH 7 to 9, nitrogenous heterocyclic buffer pH 6.5 to 9.

**10.** Reagent according to claim 9, characterised in that the zwitterionic buffer is a Good buffer.

**11.** Reagent according to claim 9 or 10, characterised in that the nitrogenous heterocyclic buffer is an imidazole buffer, pyrazole buffer or pyridine buffer.

12. Reagent according to one of claims 9 to 11, characterised in that the pH value amounts to 7.0 to 7.8 and the buffer concentration in the reconstituted, dissolved reagent is 20 to 50 mmol.

13. Reagent according to one of claims 9 to 12, characterised in that it contains superoxide dismutase.

14. Reagent according to one of claims 9 to 13, characterised in that it contains at least one additive according to claims 3, 4 or 5.

**Claims for the following Contracting States : AT, ES, GR**

1. Process for the preparation of a stabilised, NAD(P)H-dependent, soluble nitrate reductase of the assimilatory type from biological material by comminution, digestion in a neutral to weakly alkaline medium and fractionation in the presence of a complex former and of a mercaptan, characterised in that one prepares a suspension of the comminuted starting material, containing NAD(P)H-dependent nitrate reductase of the assimilatory type, in tris-tartaric acid buffer pH 7 - 8.5, adds to this soluble polyethyleneimine in such an amount that 10 to 20% of the nitrate reductase activity passes over into the precipitate obtained, separates off the precipitate and works up the supernatant according to usual biochemical fractionation methods in the above-mentioned digestion buffer, dialyses the enzyme solution obtained and lyophilises in a zwitterionic buffer.

2. Process according to claim 1, characterised in that one adds a protease inhibitor to the buffer.

3. Process according to claim 1 or 2, characterised in that one adds FAD to the buffer.

4. Process according to claim 1, characterised in that one adds sugar or sugar alcohol to the buffer.

5. Process according to one of claims 1 to 4, characterised in that to the supernatant of the polyethyleneimine precipitation one adds so much weakly alkaline anion exchanger that nitrate reductase activity still remains in the solution and that one then elutes the exchanger with increased ion concentration.

6. Process according to one of claims 1 to 5, characterised in that one chromatographs the enzyme solution over aromatic-substituted, cross-linked agarose and obtains the enzyme with a molecular weight of about 90,000 D in the case of electrophoresis in the presence of sodium dodecyl sulphate and a residual activity after three weeks at 35°C of more than 60%.

7. Process according to one of claims 1 to 6, characterised in that, before or after the anion exchanger treatment and/or the chromatography, one precipitates the enzyme with ammonium sulphate and then again dissolves in tris-tartaric acid buffer pH 7 - 8.5.

8. Reagent for the determination of nitrate, characterised by a content of nitrate reductase obtained according to claim 1, NAD(P)H, a zwitterionic buffer pH 7 to 9, nitrogenous heterocyclic buffer pH 6.5 to 9.

9. Reagent according to claim 8, characterised in that the zwitterionic buffer is a Good buffer.

10. Reagent according to claim 8 or 9, characterised in that the nitrogenous heterocyclic buffer is an imidazole buffer, pyrazole buffer or pyridine buffer.

11. Reagent according to one of claims 8 to 10, characterised in that the pH value amounts to 7.0 to 7.8 and the buffer concentration in the reconstituted, dissolved reagent is 20 to 50 mmol.

12. Reagent according to one of claims 8 to 11, characterised in that it contains superoxide dismutase.

13. Reagent according to one of claims 8 to 12, characterised in that it contains at least one additive according to claims 2, 3 or 4.

12

**Revendications**
**Revendications pour les Etats contractants suivants : BE, CH, DE FR, GB IT, LI LU, NL, SE**

1. Nitrate réductase dépendante du NAD(P)H, stabilisée et soluble du type assimilateur, caractérisée par un poids moléculaire d'environ 90000 D par électrophorèse en présence de dodécylsulfate de sodium et par une activité résiduelle après trois semaines à 35°C supérieure à 60%, que l'on peut obtenir en préparant une suspension du produit de départ broyé contenant la nitrate réductase dépendante du NAD(P)H du type assimilateur dans du tampon tris-acide tartrique pH 7-8,5, en ajoutant à celle-ci de la polyéthylèneimine en une quantité telle que 10 à 20% de l'activité nitrate réductase passent dans le précipité qui apparaît, en séparant le précipité et en traitant le surnageant dans le tampon de dissolution cité ci-dessus selon des méthodes biochimiques de fractionnement, en dialysant la solution d'enzyme obtenue et en la lyophilisant dans un tampon zwitterionique.

2. Procédé de préparation d'une nitrate réductase dépendante du NAD(P)H, stabilisée et soluble du type assimilateur à partir d'un matériau biologique par broyage, dissolution en milieu neutre à faiblement alcalin et fractionnement en présence d'un complexant et d'un mercaptan, en particulier selon la revendication 1, caractérisé en ce que l'on prépare une suspension de produit de départ broyé contenant la nitrate réductase dépendante du NAD(P)H du type assimilateur dans du tampon tris-acide tartrique pH 7-8,5, on ajoute à celle-ci de la polyéthylèneimine soluble en une quantité telle que 10 à 20% de l'activité nitrate réductase passent dans le précipité qui se forme, on sépare le précipité et on traite de surnageant dans le tampon de dissolution cité ci-dessus selon des méthodes biochimiques de fractionnement, on dialyse la solution d'enzyme obtenue et on la lyophilise dans un tampon zwitterionique.

3. Procédé selon la revendication 2, caractérisé en ce que l'on ajoute au tampon une inhibiteur de protéase.

4. Procédé selon la revendication 2 ou 3, caractérisé en ce que l'on ajoute du FAD au tampon.

5. Procédé selon la revendication 2, caractérisé en ce que l'on ajoute un sucre ou un sucre-alcool au tampon.

6. Procédé selon l'une des revendications 2 à 5, caractérisé en ce que l'on ajoute au surnageant du précipité de polyéthylèneimine une quantité d'échangeur d'anions faiblement alcalin telle qu'il reste encore une activité nitrate réductase dans la solution et en ce que l'on élue ensuite l'échangeur avec une concentration ionique plus élevée.

7. Procédé selon l'une des revendications 2 à 6, caractérisé en ce que l'on chromatographie la solution d'enzyme sur de l'agarose réticulée portant des substituants aromatiques.

8. Procédé selon l'une des revendications 2 à 7, caractérisé en ce que, avant ou après le traitement avec l'échangeur d'anions et/ou la chromatographie, on précipite l'enzyme avec du sulfate d'ammonium puis on la dissout de nouveau dans du tampon tris-acide tartrique pH 7-8,5.

9. Réactif de détermination de nitrate, caractérisé en ce qu'il contient une nitrate réductase selon la revendication 1, du NAD(P)H, un tampon zwitterionique pH 7 à 9, un tampon d'hétérocycle azoté pH 6,5 à 9.

10. Réactif selon la revendication 9, caractérisé en ce que le tampon zwitterionique est un tampon de GOOD.

11. Réactif selon la revendication 9 ou 10, caractérisé en ce que le tampon d'hétérocycle azoté est un tampon d'imidazole, de pyrazole ou de pyridine.

12. Réactif selon l'une des revendications 9 à 11, caractérisé en ce que le pH est de 7,0 à 7,8 et la concentration du tampon dans le réactif reconstitué dissous est de 20 à 50 mmoles.

13. Réactif selon l'une des revendications 9 à 12, caractérisé en ce qu'il contient de la superoxyde

EP 0 244 771 B1

dismutase.

14. Réactif selon l'une des revendications 9 à 13, caractérisé en ce qu'il contient au moins un additif selon les revendications 3, 4 ou 5.

**Revendications pour les Etats contractants suivants : AT, ES, GR**

1. Procédé de préparation d'une nitrate réductase dépendante du NAD(P)H, stabilisée et soluble du type assimilateur à partir d'un matériau biologique par broyage, dissolution en milieu neutre à faiblement alcalin et fractionnement en présence d'un complexant et d'un mercaptan, caractérisé en ce que l'on prépare une suspension de produit de départ broyé contenant la nitrate réductase dépendante du NAD-(P)H du type assimilateur dans du tampon tris-acide tartrique pH 7-8,5, on ajoute à celle-ci de la polyéthylèneimine soluble en une quantité telle que 10 à 20% de l'activité nitrate réductase passent dans le précipité qui se forme, on sépare le précipité et on traite de surnageant dans le tampon de dissolution cité ci-dessus selon des méthodes biochimiques de fractionnement, on dialyse la solution d'enzyme obtenue et on la lyophilise dans un tampon zwitterionique, et on obtient l'enzyme d'un poids moléculaire d'environ 90000 D par électrophorèse en présence de dodécylsulfate de sodium et d'une activité résiduelle après trois semaines à 35°C supérieure à 60%.

2. Procédé selon la revendication 1, caractérisé en ce que l'on ajoute au tampon une inhibiteur de protéase.

3. Procédé selon la revendication 1 ou 2, caractérisé en ce que l'on ajoute du FAD au tampon.

4. Procédé selon la revendication 1, caractérisé en ce que l'on ajoute un sucre ou un sucre-alcool au tampon.

5. Procédé selon l'une des revendications 1 à 4, caractérisé en ce que l'on ajoute au surnageant du précipité de polyéthylèneimine une quantité d'échangeur d'anions faiblement alcalin telle qu'il reste encore une activité nitrate réductase dans la solution et en ce que l'on élue ensuite l'échangeur avec une concentration ionique plus élevée.

6. Procédé selon l'une des revendications 1 à 5, caractérisé en ce que l'on chromatographie la solution d'enzyme sur de l'agarose réticulée portant des substituants aromatiques.

7. Procédé selon l'une des revendications 1 à 6, caractérisé en ce que, avant ou après le traitement avec l'échangeur d'anions et/ou la chromatographie, on précipite l'enzyme avec du sulfate d'ammonium puis on la dissout de nouveau dans du tampon tris-acide tartrique pH 7-8,5.

8. Réactif de détermination de nitrate, caractérisé en ce qu'il contient une nitrate réductase obtenue selon la revendication 1, du NAD(P)H, un tampon zwitterionique pH 7 à 9, un tampon d'hétérocycle azoté pH 6,5 à 9.

9. Réactif selon la revendication 8, caractérisé en ce que le tampon zwitterionique est un tampon de GOOD.

10. Réactif selon la revendication 8 ou 9, caractérisé en ce que le tampon d'hétérocycle azoté est un tampon d'imidazole, de pyrazole ou de pyridine.

11. Réactif selon l'une des revendications 8 à 10, caractérisé en ce que le pH est de 7,0 à 7,8 et la concentration du tampon dans le réactif reconstitué dissous est de 20 à 50 mmoles.

12. Réactif selon l'une des revendications 8 à 11, caractérisé en ce qu'il contient de la superoxyde dismutase.

13. Réactif selon l'une des revendications 8 à 12, caractérisé en ce qu'il contient au moins un additif selon les revendications 2, 3 ou 4.

14